(19) Europäisches Patentamt
European Patent Office
Office européen des brevets

(11) **EP 2 502 546 B1**

(12) **EUROPEAN PATENT SPECIFICATION**

(45) Date of publication and mention
of the grant of the patent:
**01.07.2015 Bulletin 2015/27**

(51) Int Cl.:
***A61B 1/05*** *(2006.01)*      ***A61B 1/04*** *(2006.01)*
***G06T 7/00*** *(2006.01)*

(21) Application number: **11768697.2**

(86) International application number:
**PCT/JP2011/056425**

(22) Date of filing: **17.03.2011**

(87) International publication number:
**WO 2011/129176 (20.10.2011 Gazette 2011/42)**

(54) **MEDICAL IMAGE PROCESSING APPARATUS AND MEDICAL IMAGE PROCESSING METHOD**

VORRICHTUNG ZUR VERARBEITUNG MEDIZINISCHER BILDER UND VERFAHREN ZUR VERARBEITUNG MEDIZINISCHER BILDER

APPAREIL DE TRAITEMENT D'IMAGE MÉDICALE ET PROCÉDÉ DE TRAITEMENT D'IMAGE MÉDICALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2010 JP 2010091671**

(43) Date of publication of application:
**26.09.2012 Bulletin 2012/39**

(73) Proprietor: **OLYMPUS MEDICAL SYSTEMS CORP. Tokyo 151-0072 (JP)**

(72) Inventors:
• **NISHIMURA, Hirokazu**
  **Tokyo 151-0072 (JP)**
• **TANAKA, Kenichi**
  **Tokyo 151-0072 (JP)**
• **TOMOTO, Yusuke**
  **Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

(56) References cited:
**JP-A- 2005 185 560      JP-A- 2007 175 432**
**JP-A- 2009 291 415      JP-A- 2009 297 450**
**JP-A- 2009 502 354      US-A1- 2006 050 966**

• **KARARGYRIS A ET AL: "Identification of polyps in Wireless Capsule Endoscopy videos using Log Gabor filters", LIFE SCIENCE SYSTEMS AND APPLICATIONS WORKSHOP, 2009. LISSA 2009. IEEE/NIH, IEEE, PISCATAWAY, NJ, USA, 9 April 2009 (2009-04-09), pages 143-147, XP031452064, ISBN: 978-1-4244-4292-8**
• **CHRISTOPHE SAMSON ET AL: "Multiphase Evolution and Variational Image Classification", INRIA - RAPPORT DE RECHERCHE, no. 3662, 1 April 1999 (1999-04-01), pages 1-45, XP55038661,**
• **Cheolha Pedro Lee: "Robust Image Segmentation using Active Contours: Level Set Approaches", PhD Thesis - Dept. of Electrical and Computer Engineering North Carolina State University, 1 June 2005 (2005-06-01), pages 1-146, XP55038901, Retrieved from the Internet: URL: http://repository.lib.ncsu.edu/ir/bits tream/1840.16/5246/1/etd.pdf [retrieved on 2012-09-21]**
• **TONY F CHAN ET AL: "Active Contours Without Edges", IEEE TRANSACTIONS ON IMAGE PROCESSING, IEEE SERVICE CENTER, PISCATAWAY, NJ, US, vol. 10, no. 2, 1 February 2001 (2001-02-01), XP011025732, ISSN: 1057-7149**
• **FIGUEIREDO I N ET AL: "Variational Image Segmentation for Endoscopic Human Colonic Aberrant Crypt Foci", IEEE TRANSACTIONS ON MEDICAL IMAGING, IEEE SERVICE CENTER, PISCATAWAY, NJ, US, vol. 3, no. 4, 1 April 2010 (2010-04-01), pages 998-1011, XP011297533, ISSN: 0278-0062**

EP 2 502 546 B1

- LUMINITA A VESE ET AL: "A Multiphase Level Set Framework for Image Segmentation Using the Mumford and Shah Model", INTERNATIONAL JOURNAL OF COMPUTER VISION, KLUWER ACADEMIC PUBLISHERS, BO, vol. 50, no. 3, 1 December 2002 (2002-12-01), pages 271-293, XP019216376, ISSN: 1573-1405, DOI: 10.1023/A: 1020874308076
- David Field: "Relations between the statistics of natural images and the response properties of cortical cells", J. Opt. Soc. Am. A, vol. 4, no. 12, 1 December 1987 (1987-12-01), pages 2379-2394, XP055068434,
- Jukka Kainulainen: "Clustering Algorithms: Basics and Visualization", Laboratory of Computer and Information Science T-61.195 Special Assignment 1, 1 January 2002 (2002-01-01), XP055053088, Helsinki, Finnland Retrieved from the Internet: URL:http://www.niksula.cs.hut.fi/~jkainula /pdfs/clustering.pdf [retrieved on 2013-02-12]

**Description**

Technical Field

The present invention relates to a medical image processing apparatus and a medical image processing method, and, more particularly to a medical image processing apparatus and a medical image processing method for applying image segmentation processing to a medical image obtained by picking up an image of a living tissue.

Background Art

A technique for detecting, on the basis of a calculation result of a feature value of an image obtained by picking up an image of a living tissue, an area where a predetermined object is present in the image has been conventionally used. A method of subjecting an image to image segmentation by a dynamic contour detecting method that can perform a phase change such as a Level-Set method is disclosed in Yasushi Yagi and Hideo Saito eds.: "Computer Vision Forefront Guide <1>", pp. 1-28, Advanced Communication Media Co., Ltd. (2009) (hereinafter, referred to as Non-Patent Document 1), Tony F. Chan and Luminita A. Vese: "Active Contours Without Edges", IEEE TRANSACTIONS ON IMAGE PROCESSING, VOL. 10, NO. 2, February 2001, pp. 266-277 (hereinafter, referred to as Non-Patent Document 2), and Christophe Samson, Laure Blanc-Feraud, Gilles Aubert, and Josiane Zerubia: "Multiphase Evolution and Variational Image Classification", INRIA Sophia Antipolis (1999) (hereinafter referred to as Non-Patent Document 3).

Further, for example, Japanese Patent Application Laid-Open Publication No. 2007-313119 (hereinafter, referred to as Patent Document 1) discloses a technique for calculating edge intensity of a pixel in an intra-body cavity image, calculating a correlation value between the edge intensity and a bubble model set in advance on the basis of a characteristic of a bubble image, subjecting the intra-body cavity image to image segmentation on the basis of an edge of the intra-body cavity image, and detecting a bubble area in the intra-body cavity image on the basis of the correlation value and a result of the image segmentation.

Incidentally, in recent years, with a resolution enhancement in endoscope (including capsule endoscope) systems, a spread of magnifying endoscopes, and a spread of narrow-band imaging, an importance of observation of an image of micro vessels and a mucosal microstructure such as a pit (pit opening) structure and the like has been increased.

A pattern and a distribution state of a mucosal microstructure in a living tissue is considered to be useful for a discrimination between a normal tissue and a tumor tissue and furthermore a diagnosis of a lesion such as an estimation of an invasion depth of a cancer. However, in most cases, such a diagnosis is performed on the basis of a subjective point of view mainly according to an experience and knowledge of a doctor. Accordingly, there is a desire for implementing a differential diagnosis supporting method for an evidence of a mucosal microstructure using an image analysis method, for example, as a diagnosis method not depending on a subjective point of view of a doctor. In addition, study for such a differential diagnosis supporting method has been performed as one method of Computer Aided Diagnosis (CAD).

Meanwhile, in analysis of an evidence of a mucosal microstructure in an endoscopic image, since the evidence includes wide variety of types and the evidence is complicated and image pickup conditions at the time of acquiring an endoscopic image such as an observation distance, direction and angle, an illumination light amount, and the like are not constant, it is considered to be difficult to extract a mucosal microstructure from an endoscopic image according to the evidence.

For example, the technique related to the image segmentation method and the detecting method disclosed in Patent Document 1 has a problem in that, if such a technique is applied to an image acquired by picking up an image of bubbles having a large edge intensity substantially evenly, an effective processing result can be obtained, but if it is applied to an image acquired by picking up an image of blood vessels which are likely to include a wide variety of large and small edge intensities, there is a difficulty in obtaining an effective processing result. As a result, if the technique related to the image segmentation method and the detecting method disclosed in Patent Document 1 is used for an image acquired by picking up an image of blood vessels, for example, the detection accuracy of blood vessels will be decreased.

In addition, Non-Patent Document 1 discloses the method of subjecting an image to image segmentation by a dynamic contour detecting method which is called the Level-Set method. Specifically, Non-Patent Document 1 discloses the technique which is capable of dynamically setting boundary lines which define areas in an image by performing repeated processing based on edge sizes of structural components in the image.

However, endoscopic images are likely to include, as structural components in the image, wide variety of components such as micro vessels, a pit (pit opening), and mucosal micro patterns which do not have clear edges, in addition to a shape contour and a visible vascular pattern which have clear edges. Accordingly, even if image segmentation is performed on an endoscopic image using the method disclosed in Non-Patent Document 1, a favorable result cannot necessarily be obtained for the purpose of extracting a mucosal microstructure from the endoscopic image.

In contrast, Non-Patent Document 2 discloses, as a method similar to the Level-Set method, a method for performing image segmentation on an image using a dynamic contour detecting method based on Mumford-Shah model (hereinafter, referred to as M-S method). The method disclosed in Non-Patent Document 2 is same as the above-described Level-Set method in that boundary lines defining areas are dynamically set by performing repeated processing, but different in that boundary lines can be set based on a value such as an average value of feature values in the respective areas inside and outside the boundary lines. Specifically, Non-Patent Document 2 discloses the technique for enabling image segmentation to be performed even on an image including structures which do not have clear edges by setting boundary lines such that the feature values become uniform as much as possible inside and outside the areas when the boundary lines defining the areas are dynamically set through repeated processing.

However, according to the method disclosed in Non-Patent Document 2, if the method is applied to a relative simple image such as an image in which overlapping of distributions of the feature values inside and outside the areas is few, or an image in which an area having uniform brightness exists in isolation, a favorable result can be obtained, on the other hand, if the method is applied to an endoscopic image which is likely to include a complicated mucosal microstructure and whose image pickup conditions such as an observation distance, direction and angle, an illumination light amount, and the like are likely to change constantly, a favorable result cannot necessarily be obtained. Specifically, according to the method disclosed in Non-Patent Document 2, when feature values are calculated in an endoscopic image obtained by picking up images of a plurality of micro vessels, in view of influences caused by a slight difference of depths at which the micro vessels run under the mucous membrane, a change in light amount at positions within a field of view, and a secondary light reflected from peripheral mucous membranes, the feature values are distributed in a very wide range, therefore the boundary lines of micro vessels cannot be set accurately.

In addition, Non-Patent Document 3 discloses a method in which substantially the same algorithm as disclosed in Non-Patent Document 2 is used, but the distribution of feature values for the respective areas inside and outside the boundary lines is used as a proper distribution model, and an average value $\mu$ and a standard deviation $\sigma$ (or variance $\sigma^2$ which is the square of the standard deviation) as parameters are set in advance, thereby setting the boundary lines such that the parameters of the feature values in the respective areas approximate the set parameters.

When any one of the methods disclosed in Non-Patent Documents 1 to 3 is applied to an image segmentation of an endoscopic image, it is necessary to take at at least the following points (A) to (D) into consideration. However, Non-Patent Documents 1 to 3 neither conceive of the following points (A) to (D) nor provide specific disclosures thereof.

(A) Parameters have to be set in advance for respective categories into which the areas are divided.
(B) Since the number of categories into which areas are to be divided is not known in the endoscopic image, it is unclear how many functions for dynamically setting a contour should be prepared.
(C) In addition to (B), when the method disclosed in Non-Patent Document 3 is used, it is not clear how many parameters corresponding to the number of categories should be prepared, and a problem is how to set the values of parameters. In view of the fact that accuracy of image segmentation result depends on the set parameters, appropriate parameters should be set for each image.
(D) In an endoscopic image, if taking the case of blood vessels as an example, in some cases, image segmentation has to be performed by treating the blood vessels as belonging to one category of blood vessel in histology, while treating the blood vessels as belonging to categories different from each other according to a difference in the feature values of the structural components.

The following document discloses a method for identifying polyps in Wireless Capsule endoscopy: KARARGYRIS A ET AL: "Identification of polyps in Wireless Capsule Endoscopy videos using Log Gabor filters", LIFE SCIENCE SYSTEMS AND APPLICATIONS WORKSHOP, 2009. LISSA 2009. IEEE/NIH, IEEE, PISCATAWAY, NJ, USA, 9 April 2009 (2009-04-09), pages 143-147, ISBN: 978-1-4244-4292-8.

The following document discloses a method for image segmentation: Cheolha Pedro Lee: "Robust Image Segmentation using Active Contours: Level Set Approaches", PhD Thesis - Dept. of Electrical and Computer Engineering North Carolina State University, 1 June 2005 (2005-06-01), pages 1-146, Retrieved from the Internet: URL:http://repository.lib.ncsu.edu/ir/bitstream/1840.16/5246/1/etd.pdf. US 2006/050966 A1 discloses an image processing system and an image processing method.

The following document discloses a method for image segmentation: LUMINITA A VESE ET AL: "A Multiphase Level Set Framework for Image Segmentation Using the Mumford and Shah Model", INTERNATIONAL JOURNAL OF COMPUTER VISION, KLUWER ACADEMIC PUBLISHERS, BO, vol. 50, no. 3, 1 December 2002 (2002-12-01), pages 271-293, ISSN: 1573-1405, DOI: 10.1023/A: 1020874308076.

The present invention, which is defined in the independent claims, has been achieved in view of the above-described circumstances, and an object of the present invention is to provide a medical image processing apparatus and a medical image processing method which are capable of favorably segmenting an area in which blood vessels and a mucosal microstructure such as a pit are present and an area in which bleeding, residue, and a substance such as a fur are present, as areas different from each other, in an image acquired by picking up an image of a surface of living mucosa (including an image acquired by using an endoscope or a capsule endoscope), by estimating the number of categories into which areas are to be divided for each image as a processing target and appropriately setting a parameter of a feature value for each of the categories, thereby resultantly improving detection accuracy of desired components present in the image.

Disclosure of Invention

Means for Solving the Problem

A medical image processing apparatus according to the present invention : comprises the features of claim 1.
A medical image processing method according to the present invention comprises the features of claim 6.

Brief Description of the Drawings

Fig. 1 is a diagram showing an example of a configuration of a main part of an endoscope apparatus including a medical image processing apparatus according to an embodiment of the present invention;
Fig. 2 is a diagram showing an example of a configuration of a rotating filter included in a light source device shown in Fig. 1;
Fig. 3 is a diagram showing an example of a transmission characteristic of filters included in a first filter groups shown in Fig. 2;
Fig. 4 is a diagram showing an example of a transmission characteristic of filters included in a second filter group shown in Fig. 2;
Fig. 5 is a flowchart showing an example of processing performed in a first embodiment of the present invention;
Fig. 6 is a schematic diagram showing an example of image data set as a processing target;
Fig. 7 is a diagram showing an example of an edge detection filter;
Fig. 8 is a diagram showing a case in which plural circles are set as an initial solution of an arithmetic operation related to image segmentation in the image data shown in Fig. 6;
Fig. 9 is a diagram showing an example of an arithmetic operation in progress related to image segmentation processing;
Fig. 10 is a diagram showing an example of a processing result obtained by the arithmetic operation related to the image segmentation processing;
Fig. 11 is a flowchart showing an example of processing performed in a second example of the present invention;
Fig. 12 is a diagram showing an example of a mixed normal distribution related to an occurrence frequency of a predetermined color-tone feature value in each pixel of image data; and
Fig. 13 is a diagram showing an example of two normal distributions estimated on the basis of the mixed normal distribution shown in Fig. 12.

Best Mode for Carrying Out the Invention

Embodiments of the present invention are explained below with reference to the drawings.

(First Embodiment)

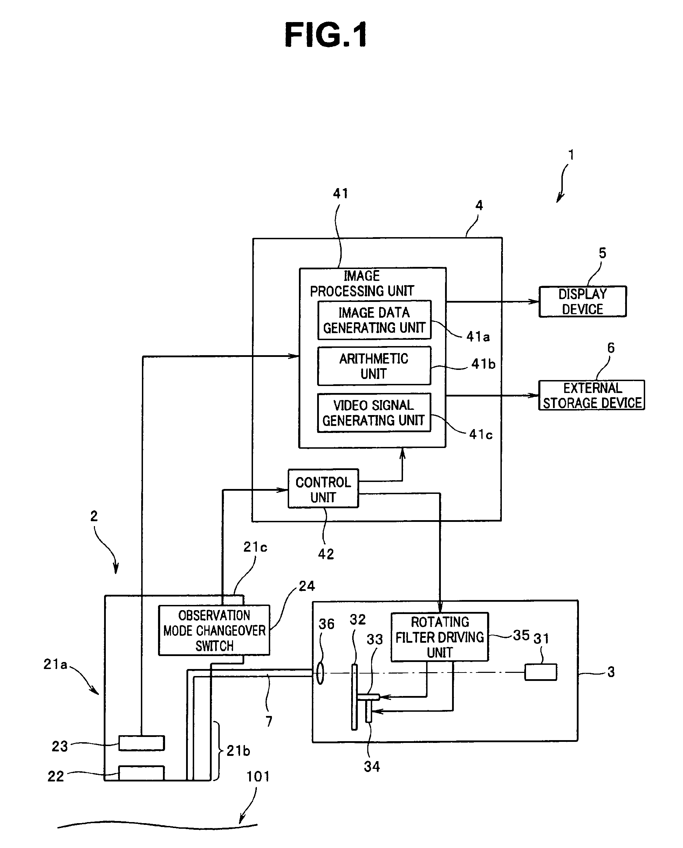

Figs. 1 to 10 relate to a first embodiment of the present invention.
An endoscope apparatus 1 includes, as shown in Fig. 1, an endoscope 2 that is inserted into a body cavity of an examinee and outputs, as a signal, an image obtained by picking up an image of a subject such as a living tissue 101 in the body cavity; a light source device 3 that emits illumination light for illuminating the living tissue 101, a processor 4 that applies various kinds of processing to the output signal from the endoscope 2, a display device 5 that displays an image corresponding to a video signal from the processor 4, and an external storage device 6 that stores an output signal corresponding to a processing result in the processor 4.
The endoscope 2 includes an insertion portion 21 a having a shape and a dimension for allowing insertion into the body cavity of the examinee, a distal end portion 21 b provided on a distal end side of the insertion portion 21a, and an operation portion 2 1 c provided on a proximal end side of the insertion portion 21a. A light guide 7 for transmitting the illumination light emitted in the light source device 3 to the distal end portion 21b is inserted through an inside of the insertion portion 21a.
One end face (a light incident end face) of the light guide 7 is detachably connected to the light source device 3. The other end face (a light exit end face) of the light guide 7 is arranged near a not-shown illumination optical system provided at the distal end portion 21 b of the endoscope 2. With such a configuration, the illumination light emitted in the light source device 3 is emitted to the living tissue 101 after passing through the light guide 7 connected to the light source device 3 and the not-shown illumination optical system provided at the distal end portion 21b.

An object optical system 22 that forms an optical image of a subject and a CCD 23 that picks up the optical image formed by the object optical system 22 and acquires an image are provided at the distal end portion 21b of the endoscope 2. An observation mode changeover switch 24 that can perform an instruction for switching an observation mode to a normal light observation mode or a narrowband light observation mode is provided in the operation portion 21c of the endoscope 2.

The light source device 3 includes a white light source 31 including a Xenon lamp, a rotating filter 32 that changes white light emitted from the white light source 31 to frame-sequential illumination light, a motor 33 that drives to rotate the rotating filter 32, a motor 34 that moves the rotating filter 32 and the motor 33 in a direction perpendicular to an emission optical path of the white light source 31, a rotating filter driving unit 35 that drives the motors 33 and 34 on the basis of control by the processor 4, and a condensing optical system 36 that condenses illumination light passed through the rotating filter 32 and supplies the illumination light to the incident end face of the light guide 7.

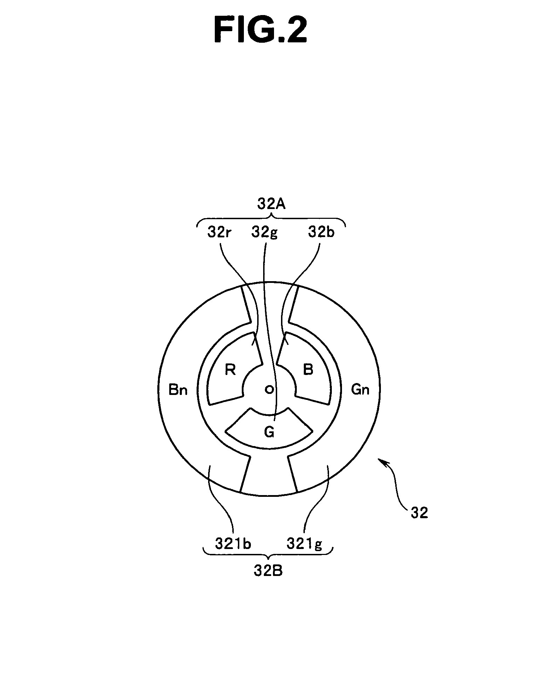

As shown in Fig. 2, the rotating filter 32 is configured in a disk shape having a rotating shaft in a center and includes a first filter group 32A including plural filters provided along a circumferential direction on an inner circumferential side and a second filter group 32B including plural filters provided along a circumferential direction on an outer circumferential side. Driving force of the motor 33 is transmitted to the rotating shaft, whereby the rotating filter 32 rotates. In the rotating filter 32, portions other than portions where the filters of the first filter group 32A and the second filter group 32B are arranged are made of a light blocking member.

The first filter group 32A includes an R filter 32r that transmits light in a wavelength band of red, a G filter 32g that transmits light in a wavelength band of green, and a B filter 32b that transmits light in a wavelength band of blue. The filters 32r, 32g, and 32b are provided along the circumferential direction on the inner circumferential side of the rotating filter 32.

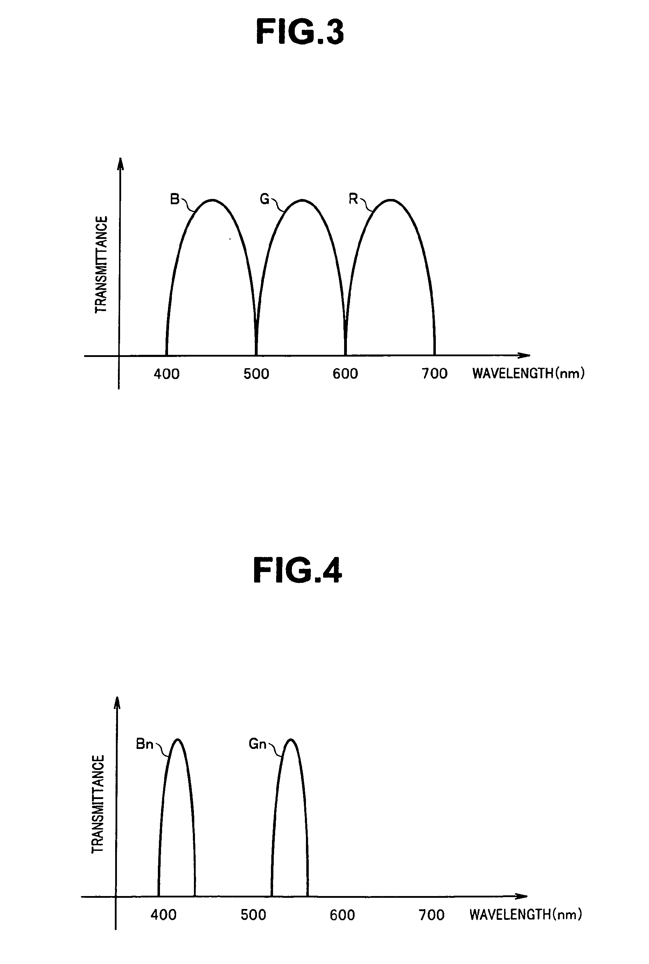

For example, as shown in Fig. 3, the R filter 32r has a configuration for mainly transmitting light of 600 nm to 700 nm (R light). For example, as shown in Fig. 3, the G filter 32g has a configuration for mainly transmitting light of 500 nm to 600 nm (G light). For example, as shown in Fig. 3, the B filter 32b has a configuration for mainly transmitting light of 400 nm to 500 nm (B light).

In other words, the white light emitted in the white light source 31 passes through the first filter group 32A, whereby wideband light for the normal light observation mode is generated.

The second filter group 32B includes a Bn filter 321b that transmits blue and narrowband light and a Gn filter 321g that transmits green and narrowband light. The filters 321b and 321g are provided along the circumferential direction on the outer circumferential side of the rotating filter 32.

For example, as shown in Fig. 4, the Bn filter 321b has a center wavelength set near 415 nm and is configured to transmit light in a narrow band compared with B light (Bn light).

For example, as shown in Fig. 4, the Gn filter 321 g has a center wavelength set near 540 nm and is configured to transmit light in a narrow band compared with G light (Gn light).

In other words, the white light emitted in the white light source 31 is dispersed through the second filter group 32B, whereby narrowband light in plural bands for the narrowband light observation mode is generated.

The processor 4 includes a function of an image processing apparatus. Specifically, the processor 4 includes an image processing unit 41 and a control unit 42. The image processing unit 41 includes an image data generating unit 41 a, an arithmetic unit 41 b, and a video signal generating unit 41 c.

The image data generating unit 41a of the image processing unit 41 applies processing such as noise removal and A/D conversion to an output signal from the endoscope 2 on the basis of control by the control unit 42 to thereby generate image data corresponding to an image obtained in the CCD 23.

The arithmetic unit 41b of the image processing unit 41 performs predetermined processing using the image data generated by the image data generating unit 41 a to thereby perform image segmentation processing for image data obtained by picking up an image of the living tissue 101. In the present embodiment, it is assumed that a blood vessel is included in the image data and image segmentation processing for distinguishing an area where the blood vessel is present from other areas in the image data is performed. Details of such image segmentation processing are explained later in detail.

The video signal generating unit 41 c of the image processing unit 41 applies processing such as gamma conversion and D/A conversion to the image data generated by the image data generating unit 41a to thereby generate and output a video signal.

When it is detected that an instruction for switching the observation mode to the normal light observation mode is performed on the basis of an instruction of the observation mode changeover switch 24, the control unit 42 applies control for causing the light source device 3 to emit wideband light for the normal light observation mode to the rotating filter driving unit 35. The rotating filter driving unit 35 causes, on the basis of control by the control unit 42, the motor 34 to operate to interpose the first filter group 32A on the emission optical path of the white light source 31 and retract the second filter group 32B from the emission optical path of the white light source 31.

When it is detected that an instruction for switching the observation mode to the narrowband light observation mode is performed on the basis of an instruction of the observation mode changeover switch 24, the control unit 42 applies control for causing the light source device 3 to emit narrowband light in plural bands for the narrowband light observation mode to the rotating filter driving unit 35. The rotating filter driving unit 35 causes, on the basis of control by the control unit 42, the motor 34 to operate to interpose the second filter group 32B on the emission optical path of the white light source 31 and retract the first filter group 32A from the emission optical path of the white light source 31.

In other words, with the configuration of the endoscope apparatus 1 explained above, when the normal light observation mode is selected, it is possible to cause the display device 5 to display an image having a color tone substantially the same as a color tone of an object seen by naked eyes (a normal light image) and (or) cause the external storage device 6 to store the image. With the configuration of the endoscope apparatus 1 explained above, when the narrowband light observation mode is selected, it is possible to cause the display device 5 to display an image with a blood vessel included in the living tissue 101 highlighted (a narrowband light image) and (or) cause the external storage device 6 to store the image.

Actions of the present embodiment are explained below.

After turning on a power supply for the units of the endoscope apparatus 1, a surgeon selects the normal light observation mode in the observation mode changeover switch 24. The surgeon inserts the endoscope 2 into a body cavity while looking at an image displayed on the display device 5 when the normal light observation mode is selected, i.e., an image having a color tone substantially the same as a color tone of an object seen by naked eyes to thereby bring the distal end portion 21 b close to an area where the living tissue 101 as an observation target is present.

When the normal light observation mode is selected in the observation mode changeover switch 24, lights of respective colors, i.e., R light, G light, and B light are sequentially emitted from the light source device 3 to the living tissue 101. Images respectively corresponding to the lights of the respective colors are acquired in the endoscope 2.

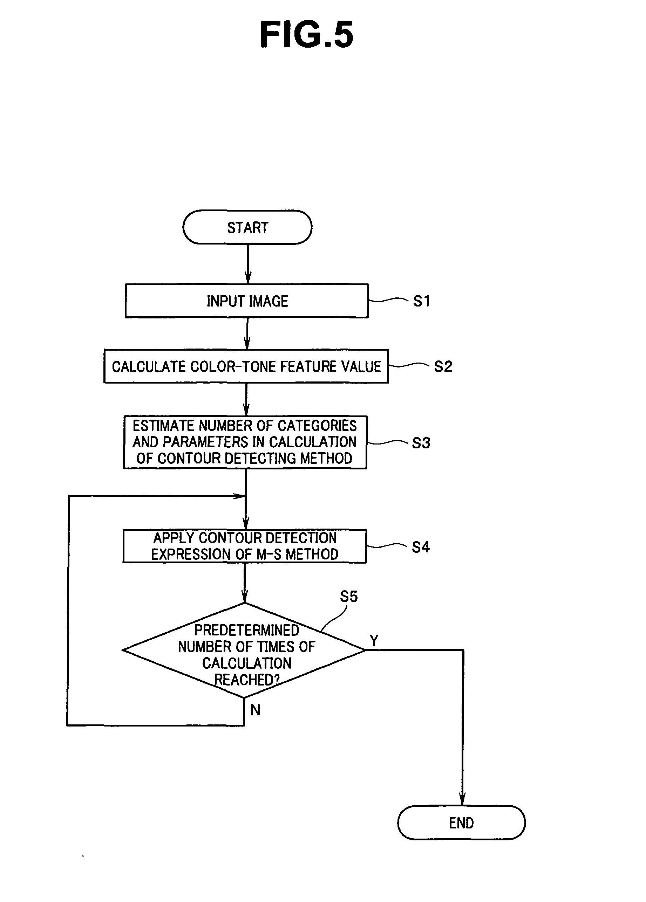

When the image corresponding to the R light, the image corresponding to the G light, and the image corresponding to the B light are inputted, the image data generating unit 41a of the image processing unit 41 generates image data respectively corresponding to the images (step S1 in Fig. 5).

In the present embodiment, it is assumed that image data that is formed by three planes for R, G, and B and includes an image size of a horizontal direction ISX and a vertical direction ISY and gradations of each of the pixels are 8 bits of 0 to 255 is generated by the image data generating unit 41 a. In the following explanation, pixel values of jth ($1 \leq j < ISX \times ISY$) pixels in the planes for R, G, and B are respectively represented as rj, gj, and bj.

The arithmetic unit 41 b of the image processing unit 41 calculates, on the basis of the image data generated by the image data generating unit 41a, for each of the pixels of the image data, a predetermined color-tone feature value used for the following processing (step S2 in Fig. 5).

More specifically, the arithmetic unit 41 b in the present embodiment calculates, for each of the pixels (in all first to ($ISX \times ISY$) pixels), a value of gj/rj, which is a ratio of a pixel value rj of a jth pixel of the R plane and a pixel value gj of a jth pixel of the G plane, as the predetermined color-tone feature value (a color-tone feature value calculated on the basis of image data generated when the normal light observation mode is selected).

In the present embodiment, the following explanation is made assuming that the pixel value $rj \neq 0$ always holds and assuming that an occurrence frequency of the predetermined color-tone feature value conforms to a normal distribution (a multivariate normal distribution).

Thereafter, the arithmetic unit 41b of the image processing unit 41 performs, using the predetermined color-tone feature value calculated in step S2 in Fig. 5, image segmentation processing for distinguishing an area where a blood vessel is present in the living tissue 101 from other areas.

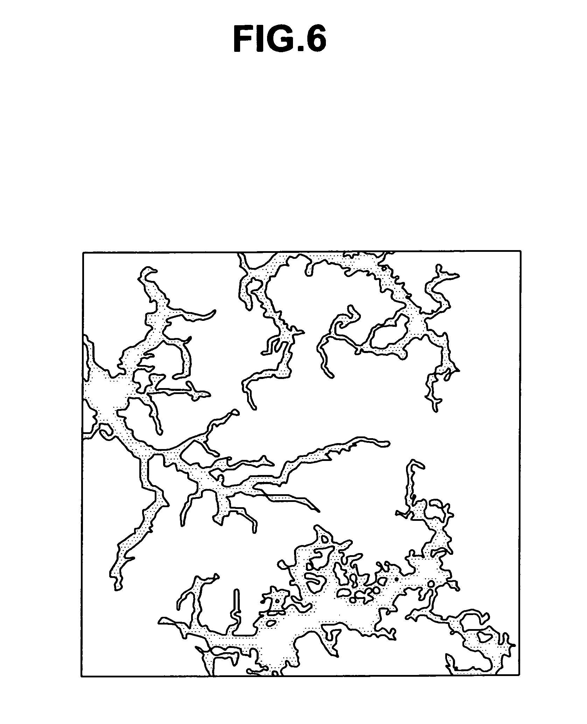

Details of the image segmentation processing in the present embodiment are explained. In the following explanation, for simplification of the explanation, it is assumed that processing is applied to schematic image data in which an area equivalent to an inside of a blood vessel (an inner side of the blood vessel) is represented as a dot pattern, an area equivalent to a background mucous membrane (an outer side of the blood vessel) is represented as white, and a boundary line of these two areas are represented as a thin solid line, for example, as shown in Fig. 6.

Fig. 6 schematically shows a blood vessel exhibiting a complicated shape called an aggregated veinlet of a tunica mucosa ventriculi observed using a magnification endoscope. According to an actual endoscopic image, not only image data including only a blood vessel and a background mucous membrane area but also image data further including various structures such as a micro vessel and a fundic pit (pit opening) that branch from the aggregated veinlet and run toward a surface layer mucous membrane is acquired. It is possible to apply a series of processing explained below by increasing the number of categories as appropriate regarding that these various structures belong to other categories different from both the blood vessel and the background mucous membrane area.

The arithmetic unit 41 b performs an arithmetic operation for estimating the number of categories and a parameter for each of the categories in an arithmetic operation of a contour detecting method employing a dynamic contour model similar to a Mumford-Shah model disclosed in the Non-Patent Document 3 (Christophe Samson, Laure Blanc-Feraud, Gilles Aubert, and Josiane Zerubia: "Multiphase Evolution and Variational Image Classification", INRIA Sophia Antipolis) (step S3 in Fig. 5). An example of such a method of estimating a parameter is explained below.

First, processing for extracting an area having clear various structural components and a background area such as an interstitial portion from an image and calculating the number of categories and a parameter for each of the categories based on feature values of these areas is explained.

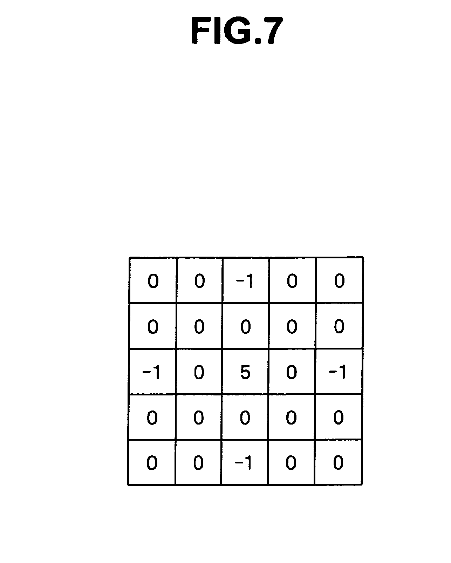

The arithmetic unit 41 b extracts, by applying a convolutional operation employing a publicly-known edge detection filter (e.g., a filter having a size $5\times5$ shown in Fig. 7) to the G plane of the schematic image data shown in Fig. 6, an area having a large edge (an area having large fluctuation in a pixel value and a long edge) and a global background area (an area rarely having an edge). Specifically, when an image after application of edge detection filtering is represented as Ga and a pixel value of a jth ($1\leq j<ISX\times ISY$) pixel in the image Ga is represented as gaj, for example, after binarizing and extracting each pixel in a range of $|gaj|>0$ (| | represents an absolute value), the arithmetic unit 41 b creates an edge map M by applying publicly-known labeling to each of linked components. The following explanation is continued assuming that, in the edge map M created in this way, labeled C ($1\leq C$) linked components are present and representing the linked components as mc ($1\leq c\leq C$).

In the image Ga obtained on the basis of a micro structure of a mucous membrane surface in a normal endoscopic image when a stain or the like is not used, since hemoglobin absorbs a wavelength component included in the G light, a pixel value of a blood vessel is relatively small with respect to a pixel value of a peripheral mucous membrane. In the image Ga, since the wavelength component included in the G light is reflected, a pixel value of a pit is relatively large with respect to the pixel value of the peripheral mucous membrane. It is possible to extract various categories by making use of such a characteristic.

On the other hand, the arithmetic unit 41 b creates a large edge map L different from the edge map M in order to extract a clear edge of a blood vessel. Specifically, the arithmetic unit 41 b creates the large edge map L by binarizing and extracting each pixel in a range of $|gaj|>Th1$ (e.g., Th1=10). The threshold Th1 is not limited to a threshold set as a fixed value and may be, for example, a threshold adaptively determined to include pixel values of higher order 20% in a histogram related to the pixel value gaj.

A reason for creating the large edge map L is as explained below.

According to the schematic image data shown in Fig. 6, only the blood vessel and the background area are present in the image. However, according to an actual endoscopic image, various blood vessels, other structural components, and noise components could be included in the endoscopic image. Therefore, when the edge map M is created, edges of the blood vessels, the structural components, and the noise components are extracted as being in the range of $|gaj|>0$. In view of such circumstances, the large edge map L different from the edge map M is created, whereby a large edge forming a closed area or a large edge included in at least a part of the closed area is specified. By performing processing explained later, it is possible to exclude a structural component and a noise component formed only by an edge having a small value, i.e., it is possible to specify only a relatively clear structural component.

Subsequently, the arithmetic unit 41b performs, by alternately referring to the edge map M and the large edge map L, processing for selecting (one or plural) linked components mc that overlap a pixel in which it is determined that a large edge is present in the large edge map L among linked components m1 to mC of the edge map M. According to such processing, it is possible to extract only (one or plural) linked components in which it is highly likely that an image of a blood vessel is picked up equivalent to an area having a large edge among the linked components of the edge map M.

Further, the arithmetic unit 41 b extracts, on the basis of a processing result of the processing, respective closed areas formed by the linked components mc determined as linked components having a large edge using, for example, a closed area detecting method disclosed in Japanese Patent Application Laid-Open Publication No. 11-003428. Representative areas Ad ($1\leq d\leq D$) extracted in this way are extracted as areas including various structural components set as targets of image segmentation in an image and areas including relatively clear structural components among the various structural components.

The arithmetic unit 41 b regards, as a global background area, a pixel group not extracted as an edge in the edge map M and extracts the pixel group.

Thereafter, the arithmetic unit 41 b repeats processing for integrating areas having values of color-tone feature values gj/rj similar to each other as one category in the extracted each representative area Ad to thereby estimate the number of all categories K ($1\leq K$) included in the schematic image data shown in Fig. 6. As such processing, it is possible to apply processing related to clustering employing a color-tone feature value and a histogram of the color-tone feature value disclosed in, for example, Japanese Patent Application Laid-Open Publication No. 2007-175432.

Subsequently, in the respective K categories obtained by the processing explained above, the arithmetic unit 41b calculates $\mu GR_i$ and $\sigma GR_i$ ($1\leq i\leq K$), which are an average and a standard deviation of values of the color-tone feature values gj/rj.

Concerning the calculation of the number of categories and a parameter, a processing target area can also be set as an area corresponding to manual operation by the surgeon or the like. In such a case, the arithmetic unit 41b only has to set an area including a structural component such as a blood vessel and a background mucous membrane area on the basis of input operation performed in a not-shown input interface, give category numbers i (1≤i≤K, K is the number of categories) to the set areas, and acquire a calculation result obtained by calculating an average and a standard deviation of the color-tone feature values gj/rj for each of the areas to which the category numbers are given.

In the estimation methods enumerated above, it is possible to improve estimation accuracy for a parameter by, before estimating a parameter, excluding a pixel having a value of a color-tone feature value that could occur redundantly in plural categories.

Concerning a pixel not suitable for calculation of a color-tone feature value such as a pixel not having sufficient brightness and a high-luminance pixel like halation, estimation of a parameter may be performed after the pixel is excluded by threshold processing.

Further, in the series of processing explained above, the processing is not limited to processing for performing estimation of a parameter on the basis of the image Ga in which the edge detection filter is applied to the G plane and may be, for example, processing for performing estimation of a parameter directly using values of the color-tone feature values gj/rj of each pixel. When estimation of a parameter is performed directly using the values of the color-tone feature values gj/rj, it is possible to reduce influences due to a magnitude of a light amount, shading, and the like, which could be a factor of exponential fluctuation of a pixel value. When estimation of a parameter is performed directly using the values of the color-tone feature values gj/rj, a series of thresholds only have to be determined as appropriate taking into account the fact that values of gj/rj in a normal endoscopic image generally fit within a range of 0<gj/rj<1.

In mucous membrane micro structures such as a blood vessel and a pit, it often occurs that, because of factors such as what kind of tissue characteristics the mucous membrane micro structures have and in how deep positions under a mucous membrane the mucous membrane micro structures are present, color tones of the mucous membrane micro structures are different from each other regardless of the fact that the mucous membrane micro structures belong to the same category as histologic structures. In such a case, for example, mucous membrane micro structures only have to be separated into plural categories such as a blood vessel 1 and a blood vessel 2 and a parameter only has to be estimated for each of the plural categories. Specifically, this can be realized by, after appropriately setting an integration criterion in performing integration of the representative areas Ad or a separation criterion for areas after the integration of the representative areas Ad is performed and estimating a final number of categories, estimating a parameter for each of the categories.

The series of processing explained above is not limited to processing for using the values of gj/rj as color-tone feature values and may be, for example, processing for appropriately selecting and using one of values of gj/rj or values of bj/gj as color-tone feature values corresponding to a category set as an extraction target.

In other words, according to the estimation methods explained above, it is possible to obtain an estimation result that the K categories in total are included in the schematic image data shown in Fig. 6. Further, according to the estimation methods explained above, it is possible to obtain, as estimation results of parameters, an average $\mu GR_i$ and a standard deviation $\sigma GR_i$ of values of the predetermined color-tone feature values gj/rj in an ith category among all the K categories.

In the following explanation, for simplification of the explanation, it is assumed that only two categories of an area equivalent to an inside of a blood vessel (an inner side of the blood vessel) and an area equivalent to a background mucous membrane such as an interstitial portion (an outer side of the blood vessel) are included in the schematic image data shown in Fig. 6 (K=2 and i=1 or 2).

On the other hand, the arithmetic unit 41 b performs an arithmetic operation related to image segmentation by applying the number of categories and the estimation results of parameters obtained in step S3 in Fig. 5 to a contour detection expression of an M-S method (step S4 in Fig. 5). In the present embodiment, as the contour detection expression, the following Equation (1) disclosed in Non-Patent Document 3 (Christophe Samson, Laure Blanc-Feraud, Gilles Aubert, and Josiane Zerubia: "Multiphase Evolution and Variational Image Classification", INRIA Sophia Antipolis) is used.

$$F_\alpha\left(\Phi_1, \cdots, \Phi_K\right) = \sum_{i=1}^{K} e_i \int_\Omega H_\alpha\left(\Phi_i\right) \frac{\left(u_0 - \mu_i\right)^2}{\sigma_i^2} dx + \sum_{i=1}^{K} \gamma_i \int_\Omega \delta_\alpha\left(\Phi_i\right) \left|\nabla\Phi_i\right| dx$$

$$+ \frac{\lambda}{2} \int_\Omega \left(\sum_{i=1}^{K} H_\alpha\left(\Phi_i\right) - 1\right)^2 dx \quad \cdot \cdot \cdot \ (1)$$

$\Phi_i$ (1≤i≤K) in Equation (1) is called distance function. A pixel at $\Phi_i$=0 is a boundary of an area. Values of $\Phi_i$ in other pixels are set according to distances from the boundary. Specifically, concerning a pixel present on an inside of the boundary (an inner side of the area), a value of $\Phi_i$ takes a positive value corresponding to a distance from the boundary. Concerning a pixel present on an outside of the boundary (an outer side of the area), the value of $\Phi_i$ takes a negative value corresponding to a distance from the boundary.

$F_\alpha(\Phi_1, ..., \Phi_K)$ in Equation (1) indicates an evaluation value obtained when an entire area $\Omega$ including all the K categories is subjected to image segmentation. In other words, it is possible to realize image segmentation of an image by calculating, through repeated processing (for example, explained later), a boundary for minimizing a value of $F_\alpha(\Phi_1, ..., \Phi_K)$.

Three terms on a right side in Equation (1) respectively mean in order a term that is smaller as a parameter of a feature value in a segmented area is closer to $\mu_i$ and $\sigma_i$, a term that is smaller as length of a boundary line is smaller (less complicated), and a term for controlling redundancy of an area and the like.

On the other hand, in image segmentation employing the M-S method, the distance function $\Phi_i$ for minimizing the evaluation value $F\alpha(\Phi_1, ..., \Phi_K)$ can be calculated through repeated processing of the following Equation (2).

$$\Phi_i^{t+1} = \Phi_i^t - dt\left(\delta_\alpha(\Phi_i)\left[e_i\frac{(u_0-\mu_i)^2}{\sigma_i^2} - \gamma_i div\left(\frac{\nabla\Phi_i}{|\nabla\Phi_i|}\right) + \lambda\left(\sum_{i=1}^{K}H_\alpha(\Phi_i)-1\right)\right]\right)$$

$$\cdots\ (2)$$

In Equation (2), t is a suffix indicating the number of times of repetition ($0 \leq t$) in the repeated processing. In Equation (2), t=0 means that a boundary is an initial solution.

In the repeated processing employing Equation (2), the arithmetic unit 41 b acquires, as an image segmentation result that converges to a final boundary, a processing result obtained when it is detected that a value of the evaluation value $F_\alpha(\Phi_1, ..., \Phi_K)$ calculated using the distance functions $\Phi_i$ respectively obtained when the number of times of repetition reaches t times and (t+1) times is 0 (or a very small change such as 0.01) or when it is detected that the number of times of repetition reaches a predetermined number of times of repetition (t = a predetermined value). In the present embodiment, for a reduction in an arithmetic operation time, the processing result obtained when the number of times of repetition reaches the predetermined number of times of repetition (distance functions $\Phi_i$ and evaluation value $F_\alpha(\Phi_1, ..., \Phi_K)$) is acquired as an image segmentation result.

In Equations (1) and (2), $\lambda$ indicates a predetermined constant (a positive real number). Specifically, in the present embodiment, $\lambda$ is set as 1.0.

In Equations (1) and (2), $e_i$ and $\gamma_i$ respectively indicate weighting coefficients of a first term and a second term of a right side of categories. Specifically, in the present embodiment, $e_i$ and $\gamma_i$ are set as $e_1=e_2=...=e_K=1.0$ and $\gamma_1=\gamma_2=...=\gamma_K=1.0$. Further, according to the present embodiment, values of these weighting coefficients are appropriately changed, whereby significance for each of the terms of the right side or each of the categories is changed. In other words, it is possible to control a convergence result (an image segmentation result) of an area boundary.

In Equations (1) and (2), $\delta_\alpha$ and $H_\alpha$ indicate functions obtained by respectively approximating a delta function of Dirac and a Heaviside distribution.

On the other hand, in step S4 in Fig. 5, the arithmetic unit 41 b updates the distance function $\Phi_i$ by substituting the predetermined color-tone feature values gj/rj (for each pixel) obtained as the processing result in step S2 in Fig. 5 in $u_0$ of Equation (2) and performs an arithmetic operation. In step S4 in Fig. 5, the arithmetic unit 41 b substitutes the number of all categories (=2) and the parameters (the average $\mu GR_i$ and the standard deviation $\alpha GR_i$ (variance $(\sigma GR_i)^2$)) estimated by the processing in step S3 in Fig. 5 in Equation (2) and performs an arithmetic operation.

Thereafter, the arithmetic unit 41 b determines whether the number of times of the arithmetic operation in step S4 in Fig. 5 reaches a predetermined number of times of the arithmetic operation (e.g., 2000 times) (step S5 in Fig. 5). When it is detected that the number of times of the arithmetic operation in step S4 in Fig. 5 does not reach the predetermined number of times of the arithmetic operation, the arithmetic unit 41b returns to step S4 in Fig. 5 and calculates the distance function $\Phi_i$ in the next number of times of repetition.

In step S5 in Fig. 5, the arithmetic unit 41b repeats the processing in step S4 until a determination result that the number of times of the arithmetic operation in step S4 in Fig. 5 reaches the predetermined times of the arithmetic operation is obtained. When a determination result that the number of times of the arithmetic operation in step S4 in Fig. 5 reaches the predetermined number of times of the arithmetic operation is obtained in step S5 in Fig. 5, the arithmetic unit 41b ends a series of processing including an arithmetic operation related to the image segmentation.

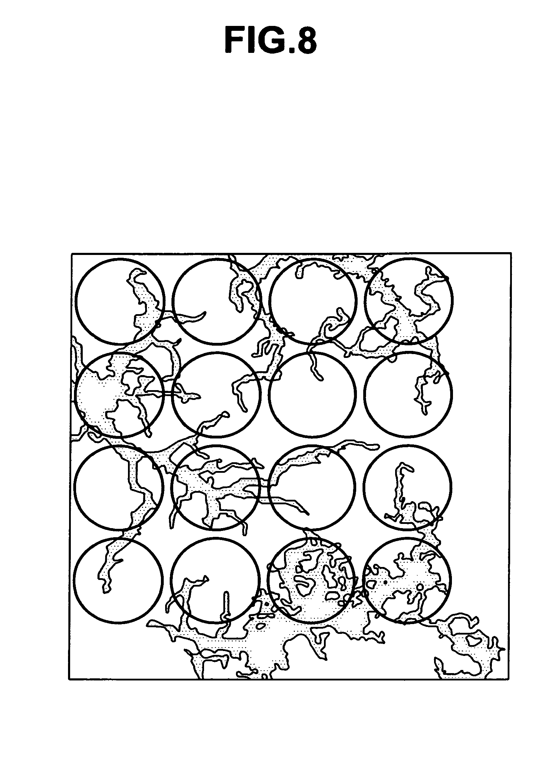

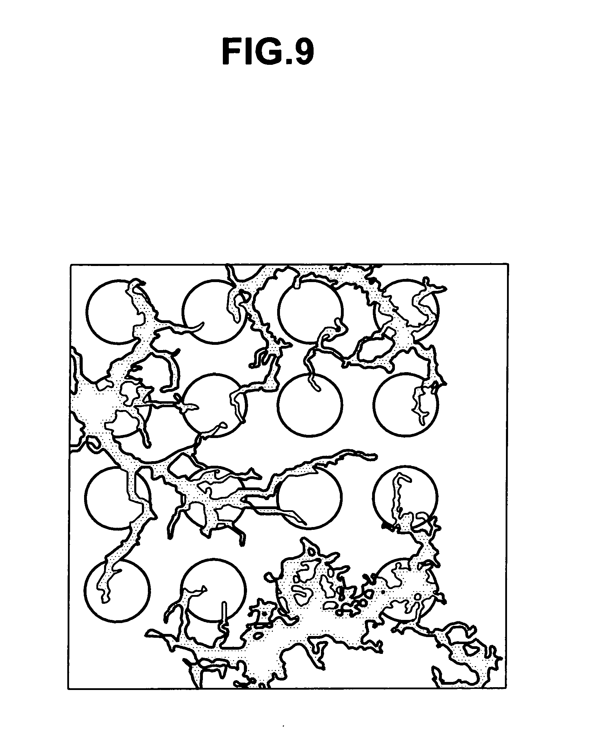

Fig. 8 is a diagram showing a case in which plural circles drawn using thick solid lines are set as an initial solution of an arithmetic operation related to image segmentation in the schematic image data shown in Fig. 6. When such an initial solution is set, for example, an arithmetic operation result shown in Fig. 9 can be obtained when the number of times of the arithmetic operation in step S4 in Fig. 5 reaches 100. When the initial solution is set, when the number of times of the arithmetic operation in step S4 in Fig. 5 reaches the predetermined number of times of the arithmetic operation, it is possible to obtain a (final) image segmentation result in which two areas of an area equivalent to an inside of a blood vessel (an inner side of the blood vessel) and an area equivalent to a background mucous membrane (an outer side of the blood vessel) are completely segmented along a boundary line indicated by a thick solid line.

In other words, according to the present embodiment, since the image segmentation processing explained above is performed using the image data generated by the image data generating unit 41a, it is possible to clearly distinguish an area where a blood vessel is present from other areas in the living tissue 101. As a result, according to the present embodiment, it is possible to improve detection accuracy of a blood vessel present in the living tissue 101 compared with the case in the past.

The image segmentation processing in the present embodiment is not limited to processing performed using an image obtained when the normal light observation mode is selected. The image segmentation processing can be performed in substantially the same manner using, for example, images obtained when the narrowband light observation mode is selected (an image corresponding to Bn light and an image corresponding to Gn light).

The image segmentation processing in the present embodiment is not limited to processing performed using, as the predetermined color-tone feature value, a value obtained by segmenting a pixel value of image data corresponding to the G light by a pixel value of image data corresponding to the R light. The image segmentation processing may be processing performed using, for example, a value obtained by segmenting a pixel value of image data corresponding to the B light by a pixel value of image data corresponding to the G light. The image segmentation processing in the present embodiment may be processing performed using, as the predetermined color-tone feature value, a pixel value of any one of image data corresponding to the R light, the G light, and the B light. Further, the image segmentation processing in the present embodiment may be processing performed using, as the predetermined color-tone feature value, a value after conversion obtained by converting pixel values of an RGB colorimetric system into an HSI colorimetric system or an L*a*b* colorimetric system.

The image segmentation processing in the present embodiment is not limited to processing using, as the predetermined color-tone feature value, only one feature value, an occurrence frequency of which conforms to a normal distribution. Plural feature values, occurrence frequencies of which respectively conform to a multivariate normal distribution, may be simultaneously used. (In this case, a variance-covariance matrix $\sum$ only has to be used as the parameter of Equation (1) instead of a variance $\sigma_i^2$.)

The image segmentation processing in the present embodiment is not limited to processing for performing image segmentation on the basis of only the two categories, i.e., the area where a blood vessel is present and the area equivalent to the background mucous membrane. The image segmentation processing may be processing for performing image segmentation on the basis of categories equivalent to elements that could be included in an endoscopic image such as a pit of a large intestine, MCE (Marginal Crypt Epithelium), a fur, and mucilage.

In the present embodiment, the image segmentation processing is not limited to processing performed assuming that an occurrence frequency of a color-tone feature value conforms to a normal distribution (a multivariate normal distribution). For example, the image segmentation processing may be performed by appropriately selecting and setting any one of a normal distribution and other probability distributions (other than the normal distribution) according to a distribution state of color-tone feature values in areas set as segmentation targets.

In the present embodiment, the processing for applying clustering to each pixel of an area having a large edge in order to estimate a parameter on the basis of a representative structural component is explained as an example. However, the processing is not limited to this and may be processing for directly performing clustering using feature values of all pixels including a background area.

(Second Example)

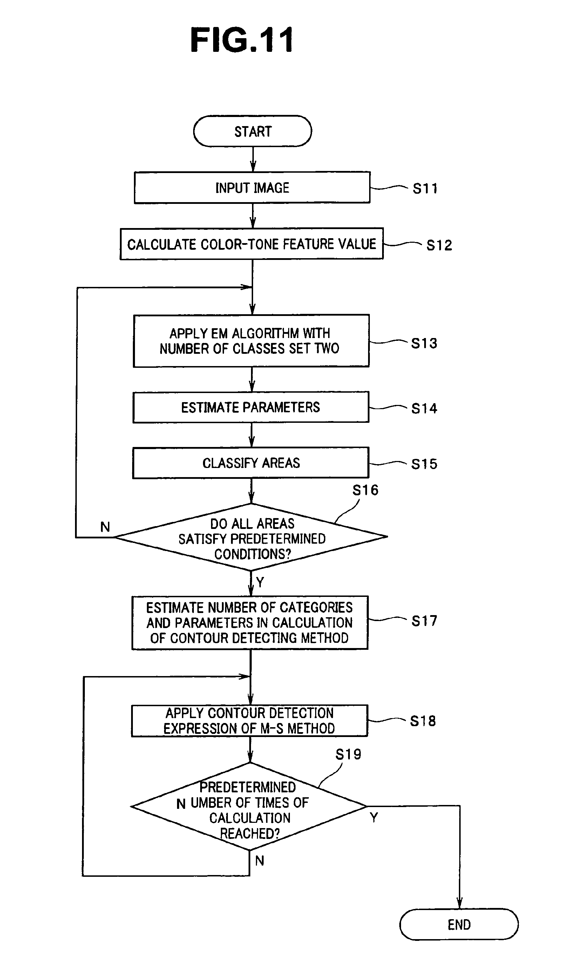

A second example of the present invention is explained next. Figs. 11 to 13 are diagrams related to the second example of the present invention. In the present example, unlike the first embodiment, a medical image processing apparatus is explained that can satisfactorily estimate the number of categories and parameters of the categories even in an image including a small number of structural components involving a clear edge, i.e., an image in which it is difficult to determine an area representing structural components on the basis of an edge.

In the present example, processing is performed using the endoscope apparatus 1 having a configuration similar to that in the first embodiment. Therefore, in the present example, detailed explanation concerning the configuration of the endoscope apparatus is omitted.

Between the present example and the first embodiment, the number of categories used for an arithmetic operation of Equation (1) and a method of estimating a parameter for each of the categories are mainly different. Therefore, in the present example, differences from the first embodiment are mainly explained and similarities to the first embodiment are explained while omitting the explanation as appropriate.

Actions of the present example are explained below.

First, after turning on a power supply for the units of the endoscope apparatus 1, a surgeon selects the normal light observation mode in the observation mode changeover switch 24. The surgeon inserts the endoscope 2 into a body cavity while looking at an image displayed on the display device 5 when the normal light observation mode is selected, i.e., an image having a color tone substantially the same as a color tone of an object seen by naked eyes to thereby bring the distal end portion 21 b close to an area where the living tissue 101 as an observation target is present.

When the normal light observation mode is selected in the observation mode changeover switch 24, lights of respective colors, i.e., R light, G light, and B light are sequentially emitted from the light source device 3 to the living tissue 101. Images respectively corresponding to the lights of the respective colors are acquired in the endoscope 2.

When the image corresponding to the R light, the image corresponding to the G light, and the image corresponding to the B light are inputted, the image data generating unit 41 a generates image data corresponding to the images (step S11 in Fig. 11).

The arithmetic unit 41 b calculates, on the basis of the image data generated in the image data generating unit 41 a, for each of pixels of the image data, values of predetermined color-tone feature values gj/rj used for the following processing (step S12 in Fig. 11).

Thereafter, the arithmetic unit 4 1 b applies an EM algorithm, the number of classes of which is set to 2, in areas that does not satisfy a predetermined condition explained later among all areas present in the schematic image data shown in Fig. 6 (in the case of first processing, an entire area of an image) (step S 13 in Fig. 11) to thereby estimate parameters (an average $\mu_i$ and a variance $\sigma_i^2$ of the predetermined color-tone feature values) in respective two classes (step S 14 in Fig. 11).

The EM algorithm is a publicly-known method that can estimate plural parameters in a mixed normal distribution. Therefore, detailed explanation concerning the EM algorithm is omitted.

In the application of the EM algorithm, it is a prerequisite that the number of classes (synonymous with the number of categories) is known. Therefore, in the present example, explanation is made assuming that the EM algorithm is applied to processing for estimating the number of categories present in an image and parameters of the categories by repeating classification in the two classes a sufficient number of times and integrating obtained results.

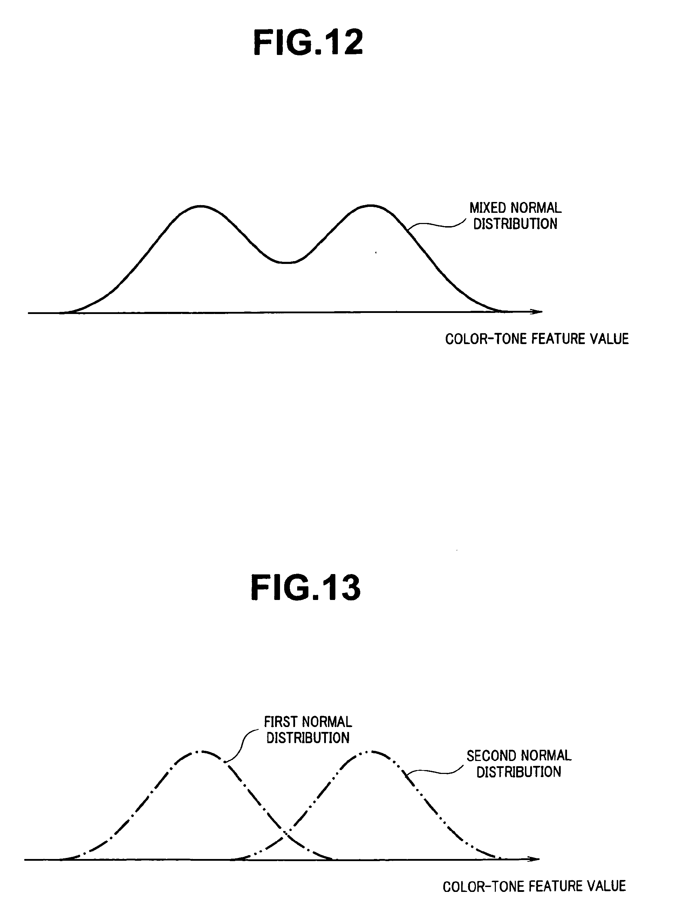

When it is a prerequisite that an occurrence frequency of a predetermined color-tone feature value (e.g., a value obtained by segmenting a pixel value of image data corresponding to the G light by a pixel value of image data corresponding to the R light) in the each pixel of the image data generated by the image data generating unit 41 a conforms to a normal distribution, a mixed normal distribution obtained by combining normal distributions of two classes is, for example, as shown in Fig 12.

The arithmetic unit 41b repeatedly performs arithmetic operations related to an E step and an M step of the EM algorithm until an arithmetic operation stop condition is satisfied (e.g., improvement of likelihood of the two classes is equal to smaller than a threshold; specifically, the threshold only has to be set to 0.01) to thereby respectively estimate parameters (an average and a variance of predetermined color-tone feature values) for specifying a first normal distribution in which a peak of a frequency is a relatively low value and parameters (an average and a variance of predetermined color-tone feature values) for specifying a second normal distribution in which a peak of a frequency is a relatively high value (step S 13 and step S 14 in Fig. 11). The first normal distribution and the second normal distribution are, for example, as shown in Fig. 13.

Subsequently, the arithmetic unit 41 b discriminates, using a normal distribution by the parameters obtained as a result of the arithmetic operation in step S 14 in Fig. 11, to which of the first and second normal distributions values of color-tone feature values gj/rj of the each pixel in the schematic image data shown in Fig. 6 are close and gives a label 1 or 2 representing a class (a category) to the each pixel (step S15 in Fig. 11). Specifically, the arithmetic unit 4 1 b determines, using, for example, a publicly-known Euclidian distance or Mahalanobis distance (when a multidimensional feature value is used), to which of average values $\mu p$ (p=1 or 2) in the first and second normal distributions the values of the color-tone feature values gj/rj of the each pixel are close.

According to the processing explained above, the each pixel in the schematic image data shown in Fig. 6 is classified into the class (the category) 1 or 2 on the basis of the values of the predetermined color-tone feature values gj/rj.

Subsequently, the arithmetic unit 41 b determines whether the series of processing in steps S 13 to S 15 is applied again to the pixel groups classified into the respective classes (the categories) 1 and 2 obtained as a result of the processing in step S 15 in Fig. 11 (step S16 in Fig. 11).

According to the first classification processing in step S15 in Fig. 11, the each pixel included in the image is basically classified into the two classes (categories). However, the number of classes (categories) corresponding to structural components actually included in the image or the number of classes (categories) optimum in performing the image segmentation processing is unknown unless separately designated. Therefore, in a classification result obtained by the first classification processing in step S15 in Fig. 11, it is likely that the pixel groups are classified into the two classes (categories) while including pixel groups that should originally be classified into different class (category).

Therefore, in step S16 in Fig. 11, the arithmetic unit 41b according to the present example performs processing for determining, according to whether a predetermined condition is satisfied, whether pixel groups (areas) to which the labels representing the classes (categories) 1 and 2 are given should be further segmented into plural classes (categories). Specifically, for example, when a difference in average values $\mu p$ between two pixel groups (areas) to which the labels representing the classes (categories) 1 and 2 are given is equal to or smaller than a threshold (e.g., the threshold is set to 0.1), when the number of pixels included in any one of the pixel groups (the areas) obtained as a classification result is equal to or smaller than a threshold (e.g., the threshold is set to 0.1×ISX×ISY), or when the number of times of repetition of the series of processing in steps S 13 to S 15 reaches a predetermined number of times, the arithmetic unit 41 b determines that the predetermined condition is satisfied. When at least one of the pixel groups (the areas) to which the labels representing the classes (the categories) 1 and 2 are given does not satisfy the predetermined condition, the arithmetic unit 41 b repeatedly performs processing for applying the series of processing in steps S13 to S 15 again to the pixel groups (the areas) that do not satisfy the predetermined condition to thereby further classify the pixel groups (the areas) into two classes (categories).

On the other hand, when all the areas (all the pixel groups) obtained as the result of the processing in step S 15 in Fig. 11 satisfy the predetermined condition, the arithmetic unit 41b estimates the number of all categories used for the arithmetic operation of Equation (1) by integrating areas where distribution states of predetermined color-tone feature values (gj/rj) partially overlap each other among all the areas or excluding the areas where the distribution states of the predetermined color-tone feature values (gj/rj) partially overlap each other (step S 17 in Fig. 11). The arithmetic unit 41 b estimates, as parameters used for the arithmetic operation of Equation (1), a calculation result obtained by calculating an average and a variance of the predetermined color-tone feature values (gj/rj) for each of the categories (step S 17 in Fig. 11).

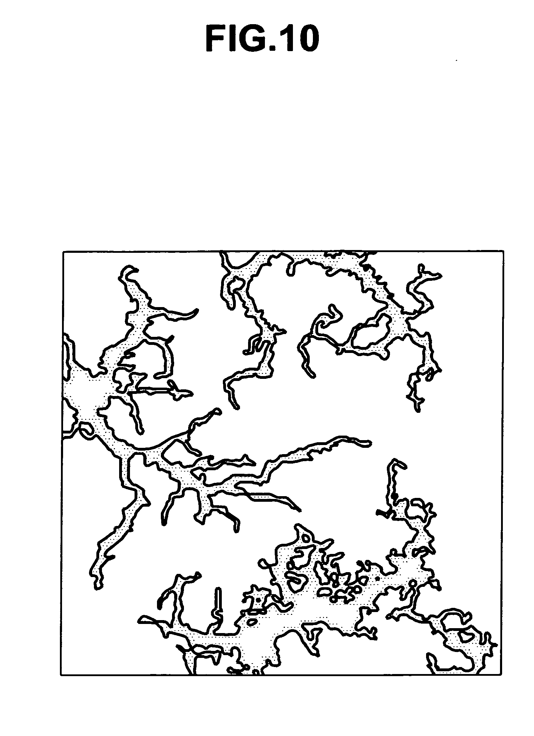

It is assumed that, according to the processing in step S17 in Fig. 11, an estimation result that only two categories of an area equivalent to an inside of a blood vessel (an inner side of the blood vessel) and an area equivalent to a background mucous membrane such as an interstitial portion (an outer side of the blood vessel) are included in the schematic image data shown in Fig. 6. When such an estimation result is obtained, the arithmetic unit 41b can obtain, by performing the same processing as the processing in steps S4 and S5 in Fig. 5 explained in the first embodiment as processing in steps S 18 and S 19 in Fig. 11, a (final) image segmentation result in which two areas of the area equivalent to the inside of the blood vessel (the inner side of the blood vessel) and the area equivalent to the background mucous membrane (the outer side of the blood vessel) are completely segmented along a boundary line indicated by a thick solid line, for example, as shown in Fig. 10.

In other words, according to the present example since the image segmentation processing explained above is performed using the image data generated by the image data generating unit 41a, it is possible to clearly distinguish an area where a blood vessel is present from other areas in the living tissue 101. As a result, according to the present example, it is possible to improve detection accuracy of a blood vessel present in the living tissue 101 compared with the case in the past.

After the series of processing in steps S 13 to S 15 in Fig. 11 is repeatedly performed, when it is determined in step S16 in Fig. 11 that the predetermined condition is satisfied, it is likely to obtain a processing result in which a pixel group that should originally be classified into the same category is excessively segmented. In such a case, areas having close values of parameters may be integrated with each other again by, for example, applying the processing related to clustering disclosed in Japanese Patent Application Laid-Open Publication No. 2007-175432 to the processing result before the processing in step S 17 in Fig. 11 is performed.

In the embodiments explained above, the arithmetic operation is performed using the estimation results of the average and the variance of the predetermined color-tone feature values as the parameters of Equation (1). However, it is also possible to perform the image segmentation processing according to a method other than this method. Specifically, for example, according to the contour detecting method disclosed in " Active Contours Without Edges" of IEEE TRANSACTIONS ON IMAGE PROCESSING, VOL. 10, NO. 2 (February 2001), it is possible to obtain an image segmentation processing result substantially the same as those in the embodiments explained above by performing the arithmetic operation using only the estimation result of the average of the predetermined color-tone feature values as a parameter.

The series of processing explained in the embodiments is not limited to processing applied to an endoscopic image and may be, for example, processing applied to an image obtained by a capsule endoscope or processing applied to various medical images such as monochrome images.

In the embodiments explained above, the processing for using color-tone feature values as feature values is explained. In other examples useful for understanding the present invention, processing may be performed by appropriately selecting feature values other than the color-tone feature values according to structural components set as segmentation targets or appropriately combining and using the color-tone feature values and the other feature values. In one example useful for understanding the present invention, as the other feature values, structural feature values such as a Gabor features obtained by applying a publicly-known Gabor filter to an image may be used.

In the embodiments explained above, the processing may be performed using color-tone feature values calculated for each pixel or may be performed using color-tone feature values calculated for each small area including plural pixels (e.g., 4x4 pixels).

The present invention is not limited to the embodiments explained above. It goes without saying that various modifications and applications are possible without departing from the scope of the claims.

## Claims

1. A medical image processing apparatus (4) capable of performing image segmentation processing for a medical image, comprising:

   a structural component area extracting unit configured to extract areas corresponding to structural components included in the medical image;
   a feature value calculating unit configured to calculate at least one color-tone feature value or structural feature value from each of the areas extracted by the structural component area extracting unit;
   a category estimating unit configured to estimate, on the basis of the at least one color-tone feature value or structural feature value calculated by the feature value calculating unit, plural categories corresponding to each of the areas extracted by the structural component area extracting unit;
   a parameter estimating unit configured to estimate, on the basis of the at least one color-tone feature value or structural feature value calculated by the feature value calculating unit, parameters for each of areas of the medical image respectively classified into the categories estimated by the category estimating unit, the parameters being the average of the at least one color-tone feature value or structural feature value; and
   an image segmenting unit configured to perform an arithmetic operation related to image segmentation by applying the number of categories and the estimation results of the parameters to a contour detection expression of a dynamic contour detecting method based on a Mumford-Shah model, perform a repeated operation using the contour detection expression until a predetermined condition is satisfied, and perform the image segmentation processing for the medical image based on a result of the repeated operation when the predetermined condition is satisfied,
   **characterized by** the parameters being also the standard deviation or the variance of the at least one color-tone feature value or structural feature value,
   wherein the structural component area extracting unit is configured to perform the following operations:

   - creating an edge map (M), by binarizing the result of edge detection filtering using a first threshold value and by applying labeling to each of the linked components;
   - creating a large edge map (L) by binarizing the result of edge detection filtering using a second threshold value higher than the first threshold value, whereby a large edge forming a closed area or a large edge included in at least a part of a closed area is specified;
   - selecting, among the linked components of the edge map (M), linked components that overlap a pixel in which it is determined that a large edge of the large edge map (L) is present;
   - extracting respective closed areas formed by the linked components determined as linked components having a large edge using a closed area detecting method to obtain representative areas as areas including various structural components set as targets for the image segmenting unit; and
   - defining, as a global background area, a pixel group not extracted as an edge in the edge map (M).

2. The medical image processing apparatus (4) according to claim 1, wherein
   the category estimating unit is configured to apply clustering processing to the plural categories on the basis of a distribution state of the at least one color-tone feature value or structural feature value calculated by the feature value calculating unit, and
   the image segmenting unit is configured to define a parameter estimated for each of areas respectively classified into categories estimated by a processing result of the clustering processing in the parameter estimating unit.

3. The medical image processing apparatus (4) according to claim 1, wherein the medical image is an endoscopic image.

4. The medical image processing apparatus (4) according to claim 1, wherein at least one of a blood vessel and a pit structure is included in the categories estimated by the category estimating unit.

5. The medical image processing apparatus (4) according to claim 1, wherein the image segmenting unit is configured to determine that the predetermined condition is satisfied, if fluctuation in an evaluation value calculated using the contour detection expression is substantially eliminated, or if the number of times of the repeated operation has reached a predetermined number of times.

6. A medical image processing method for performing image segmentation processing for a medical image, the method executing:

a structural component area extracting step in which a structural component area extracting unit extracts areas corresponding to structural components included in the medical image;

a feature value calculating step (S2) in which a feature value calculating unit calculates at least one color-tone feature value or structural feature value from each of the areas extracted by the structural component area extracting step;

a category estimating step (S3) in which a category estimating unit estimates, on the basis of the at least one color-tone feature value or structural feature value calculated by the feature value calculating step, plural categories corresponding to each of the areas extracted by the structural component area extracting step;

a parameter estimating step (S3) in which a parameter estimating unit estimates, on the basis of the at least one color-tone feature value or structural feature value calculated by the feature value calculating step, parameters for each of areas of the medical image respectively classified into the categories estimated by the category estimating step, the parameters being the average of the at least one color-tone feature value or structural feature value; and

an image segmenting step (S4) in which an image segmenting unit performs an arithmetic operation related to image segmentation by applying the number of categories and the estimation results of the parameters to a contour detection expression of a dynamic contour detecting method based on a Mumford-Shah model, performs a repeated operation using the contour detection expression until a predetermined condition is satisfied, and performs the image segmentation processing for the medical image based on a result of the repeated operation when the predetermined condition is satisfied,

**characterized by** the parameters being also the standard deviation or the variance of the at least one color-tone feature value or structural feature value,

wherein the structural component area extracting step is defined to perform the following sub-steps:

- creating an edge map (M), by binarizing the result of edge detection filtering using a first threshold value and by applying labeling to each of the linked components;
- creating a large edge map (L) by binarizing the result of edge detection filtering using a second threshold value higher than the first threshold value, whereby a large edge forming a closed area or a large edge included in at least a part of the closed area is specified;
- selecting, among the linked components of the edge map (M), linked components that overlap a pixel in which it is determined that a large edge of the large edge map (L) is present;
- extracting respective closed areas formed by the linked components determined as linked components having a large edge using a closed area detecting method to obtain representative areas as areas including various structural components set as targets for the image segmenting step; and
- defining, as a global background area, a pixel group not extracted as an edge in the edge map (M).

7. The medical image processing method according to claim 6, wherein
in the category estimating step, clustering processing is applied to the plural categories on the basis of a distribution state of the at least one color-tone feature value or structural feature value calculated by the feature value calculating step, and
the image segmenting step defines a parameter estimated for each of areas respectively classified into categories estimated by a processing result of the clustering processing in the parameter estimating step.

8. The medical image processing method according to claim 6, wherein the medical image is an endoscopic image.

9. The medical image processing method according to claim 6, wherein at least one of a blood vessel and a pit structure is included in the categories estimated by the category estimating step.

10. The medical image processing method according to claim 7, wherein the image segmenting step determines that the predetermined condition is satisfied, if fluctuation in an evaluation value calculated using the contour detection expression is substantially eliminated, or if the number of times of the repeated operation has reached a predetermined number of times.

**Patentansprüche**

1. Vorrichtung zur Verarbeitung medizinischer Bilder (4), die dazu ausgebildet ist, eine Bildsegmentierungsverarbeitung für ein medizinisches Bild auszuführen, mit:

   einer Strukturelementbereich-Extraktionseinheit, die dazu ausgebildet ist, Bereiche zu extrahieren, die in dem medizinischen Bild vorliegenden Strukturelementen entsprechen;

   einer Merkmalswert-Berechnungseinheit, die dazu ausgebildet ist, von jedem der durch die Strukturelementbereich-Extraktionseinheit extrahierten Bereiche zumindest einen Farbtonmerkmalswert oder Strukturmerkmalswert zu berechnen;

   einer Kategorie-Bewertungseinheit, die dazu ausgebildet ist, auf der Grundlage des wenigstens einen von der Merkmalswert-Berechnungseinheit berechneten Farbtonmerkmalswerts oder Strukturmerkmalswerts mehrere Kategorien zu bewerten, die jedem der von der Strukturelementbereich-Extraktionseinheit extrahierten Bereiche entsprechen;

   einer Parameter-Bewertungseinheit, die dazu ausgebildet ist, auf der Grundlage des wenigstens einen von der Merkmalswert-Berechnungseinheit berechneten Farbtonmerkmalswerts oder Strukturmerkmalswerts für jeden der Bereiche des medizinischen Bildes, die jeweils in die von der Kategorie-Bewertungseinheit bewerteten Kategorien eingestuft werden, Parameter zu bewerten, wobei die Parameter der Mittelwert des wenigstens einen Farbtonmerkmalswerts oder Strukturmerkmalswerts sind; und

   einer Bildsegmentierungseinheit, die dazu ausgebildet ist, eine mit der Bildsegmentierung in Bezug stehende Rechenoperation auszuführen durch Anwendung der Anzahl von Kategorien und der Bewertungsergebnisse der Parameter auf einen Konturerfassungsausdruck eines dynamischen, auf einem Mumford-Shah Modell basierenden Konturerfassungsverfahrens, eine Wiederholungsoperation auszuführen unter Verwendung des Konturerfassungsausdrucks bis eine vorgegebene Bedingung erfüllt ist, und die Bildsegmentierungsverarbeitung für das medizinisches Bild auszuführen, basierend auf dem Ergebnis der Wiederholungsoperation, wenn die vorgegebene Bedingung erfüllt ist,

   **dadurch gekennzeichnet, dass** die Parameter auch aus der Standardabweichung oder der Varianz des wenigstens einen Farbtonmerkmalswerts oder Strukturmerkmalswerts bestehen,

   wobei die Strukturelementbereich-Extraktionseinheit dazu ausgebildet ist, die folgenden Operationen durchzuführen:

   - Erstellen eines Kantenbilds (M) durch Binarisierung des Ergebnisses des Filterns der Kantendetektion unter Verwendung eines ersten Schwellenwerts und durch Anbringen einer Etikettierung an jedem der verbundenen Elemente;
   - Erstellen eines großen Kantenbilds (L) durch Binarisierung des Ergebnisses des Filterns der Kantendetektion unter Verwendung eines zweiten Schwellenwerts, der höher ist als der erste Schwellenwert, wodurch eine große, einen geschlossenen Bereich bildende Kante oder eine große, in wenigstens einem Teil eines geschlossenen Bereichs beinhaltete Kante festgelegt wird;
   - Auswählen, unter den verbundenen Elementen des Kantenbilds (M), von verbundenen Elementen, die ein Pixel überlappen, in dem bestimmt wird, dass eine große Kante des großen Kantenbilds (L) vorhanden ist;
   - Extrahieren von jeweiligen geschlossenen Bereichen, die durch die verbundenen Elemente gebildet werden, welche als eine große Kante aufweisende, verbundene Elemente bestimmt werden, unter Verwendung eines Verfahrens zum Erfassen von geschlossenen Bereichen, um repräsentative Bereiche zu erhalten als Bereiche, die verschiedene als Ziele für die Bildsegmentierungseinheit festgelegte Strukturelemente beinhalten; und
   - Bestimmen einer Pixelgruppe, die nicht als Kante in dem Kantenbild (M) extrahiert ist, als allgemeinen Hintergrundbereich.

2. Vorrichtung zur Verarbeitung medizinischer Bilder (4) nach Anspruch 1, bei der
   die Kategorie-Bewertungseinheit dazu ausgebildet ist, die Mehrzahl von Kategorien einer Clusterverarbeitung zu unterziehen, auf der Grundlage eines Verteilungszustands des wenigstens einen von der Merkmalswert-Berechnungseinheit berechneten Farbtonmerkmalswerts oder Strukturmerkmalswerts, und
   die Bildsegmentierungseinheit dazu ausgebildet ist, einen Parameter zu definieren, der in der Parameter-Bewertungseinheit für jeden der Bereiche bewertet wird, die jeweils in durch ein Verarbeitungsergebnis der Clusterverarbeitung bewertete Kategorien eingestuft werden.

3. Vorrichtung zur Verarbeitung medizinischer Bilder (4) nach Anspruch 1, bei der das medizinische Bild ein Endoskopbild ist.

**4.** Vorrichtung zur Verarbeitung medizinischer Bilder (4) nach Anspruch 1, bei der ein Blutgefäß und/oder eine Oberflächenstruktur in die von der Kategorie-Bewertungseinheit bewerteten Kategorien einbezogen wird.

**5.** Vorrichtung zur Verarbeitung medizinischer Bilder (4) nach Anspruch 1, bei der die Bildsegmentierungseinheit dazu ausgebildet ist, zu bestimmen, dass die vorgegebene Bedingung erfüllt ist, wenn Schwankungen in einem unter Verwendung des Konturerfassungsausdrucks berechneten Auswertungswert im Wesentlichen beseitigt sind, oder wenn die Anzahl von Malen der Wiederholungsoperation eine vorgegebene Anzahl von Malen erreicht hat.

**6.** Verfahren zur Verarbeitung medizinischer Bilder zum Ausführen einer Bildsegmentierungsverarbeitung für ein medizinisches Bild, wobei das Verfahren die folgenden Schritte umfasst:

Ausführen eines Strukturelementbereich-Extraktionsschritts, bei dem eine Strukturelementbereich-Extraktionseinheit Bereiche extrahiert, die in dem medizinischen Bild vorliegenden Strukturelementen entsprechen;
Ausführen eines Merkmalswert-Berechnungsschritts (S2), bei dem eine Merkmalswert-Berechnungseinheit von jedem der durch den Strukturelementbereich-Extraktionsschritt extrahierten Bereiche zumindest einen Farbtonmerkmalswert oder Strukturmerkmalswert berechnet;
Ausführen eines Kategorie-Bewertungsschritts (S3), bei dem eine Kategorie-Bewertungseinheit auf der Grundlage des wenigstens einen durch den Merkmalswert-Berechnungsschritt berechneten Farbtonmerkmalswerts oder Strukturmerkmalswerts mehrere Kategorien bewertet, die jedem der durch den Strukturelementbereich-Extraktionsschritt extrahierten Bereiche entsprechen;
Ausführen eines Parameter-Bewertungsschritts (S3), bei dem eine Parameter-Bewertungseinheit auf der Grundlage des wenigstens einen durch den Merkmalswert-Berechnungsschritt berechneten Farbtonmerkmalswerts oder Strukturmerkmalswerts für jeden der Bereiche des medizinischen Bildes, die jeweils in die durch den Kategorie-Bewertungsschritt bewerteten Kategorien eingestuft werden, Parameter bewertet, wobei die Parameter der Mittelwert des wenigstens einen Farbtonmerkmalswerts oder Strukturmerkmalswerts sind; und
Ausführen eines Bildsegmentierungsschritts (S4), bei dem eine Bildsegmentierungseinheit eine mit der Bildsegmentierung in Bezug stehende Rechenoperation ausführt durch Anwendung der Anzahl von Kategorien und der Bewertungsergebnisse der Parameter auf einen Konturerfassungsausdruck eines dynamischen, auf einem Mumford-Shah Modell basierenden Konturerfassungsverfahrens, eine Wiederholungsoperation ausführt unter Verwendung des Konturerfassungsausdrucks bis eine vorgegebene Bedingung erfüllt ist, und die Bildsegmentierungsverarbeitung für das medizinisches Bild ausführt, basierend auf einem Ergebnis der Wiederholungsoperation, wenn die vorgegebene Bedingung erfüllt ist,
**dadurch gekennzeichnet, dass** die Parameter auch aus der Standardabweichung oder der Varianz des wenigstens einen Farbtonmerkmalswerts oder Strukturmerkmalswerts bestehen,
wobei die Strukturelementbereich-Extraktionsschritt so definiert ist, dass er die folgenden Teilschritte ausführt:

- Erstellen eines Kantenbilds (M) durch Binarisierung des Ergebnisses des Filterns der Kantendetektion unter Verwendung eines ersten Schwellenwerts und durch Anbringen einer Etikettierung an jedem der verbundenen Elemente;
- Erstellen eines großen Kantenbilds (L) durch Binarisierung des Ergebnisses des Filterns der Kantendetektion unter Verwendung eines zweiten Schwellenwerts, der höher ist als der erste Schwellenwert, wodurch eine große, einen geschlossenen Bereich bildende Kante oder eine große, in wenigstens einem Teil des geschlossenen Bereichs beinhaltete Kante festgelegt wird;
- Auswählen, unter den verbundenen Elementen des Kantenbilds (M), von verbundenen Elementen, die ein Pixel überlappen, in dem bestimmt wird, dass eine große Kante des großen Kantenbilds (L) vorhanden ist;
- Extrahieren von jeweiligen geschlossenen Bereichen, die durch die verbundenen Elementen gebildet werden, welche als eine große Kante aufweisende, verbundene Elemente bestimmt werden, unter Verwendung eines Verfahrens zum Erfassen von geschlossenen Bereichen, um repräsentative Bereiche zu erhalten als Bereiche, die verschieden als Ziele für den Bildsegmentierungsschritt festgelegte Strukturelemente beinhalten; und
- Bestimmen einer Pixelgruppe, die nicht als Kante in dem Kantenbild (M) extrahiert ist, als allgemeinen Hintergrundbereich.

**7.** Verfahren zur Verarbeitung medizinischer Bilder nach Anspruch 6, bei dem
bei dem Kategorie-Bewertungsschritt die Mehrzahl von Kategorien einer Clusterverabeitung unterzogen werden, auf der Grundlage eines Verteilungszustands des wenigstens einen durch den Merkmalswert-Berechnungsschritt berechneten Farbtonmerkmalswerts oder Strukturmerkmalswerts, und
der Bildsegmentierungsschritt einen Parameter definiert, der in dem Parameter-Bewertungsschritt für jeden der Bereiche bewertet wird, die jeweils in durch ein Verarbeitungsergebnis der Clusterverarbeitung bewertete Kategorien eingestuft werden.

8. Verfahren zur Verarbeitung medizinischer Bilder nach Anspruch 6, bei dem das medizinische Bild ein Endoskopbild ist.

9. Verfahren zur Verarbeitung medizinischer Bilder nach Anspruch 6, bei dem ein Blutgefäß und/oder eine Oberflächenstruktur in die durch den Kategorie-Bewertungsschritt bewerteten Kategorien einbezogen wird.

10. Verfahren zur Verarbeitung medizinischer Bilder nach Anspruch 7, bei dem der Bildsegmentierungsschritt bestimmt, dass die vorgegebene Bedingung erfüllt ist, wenn Schwankungen in einem unter Verwendung des Konturerfassungsausdrucks berechneten Auswertungswert im Wesentlichen beseitigt sind, oder wenn die Anzahl von Malen der Wiederholungsoperation eine vorgegebene Anzahl von Malen erreicht hat.

**Revendications**

1. Appareil (4) de traitement d'image médicale capable de réaliser un traitement de segmentation d'image pour une image médicale, comprenant :

    une unité d'extraction de zones de composantes de structure configurée pour extraire des zones correspondant à des composantes de structure contenues dans l'image médicale ;
    une unité de calcul de valeur de caractéristique configurée pour calculer au moins une valeur de caractéristique de tonalité de couleur ou une valeur de caractéristique de structure à partir de chacune des zones extraites par l'unité d'extraction de zones de composantes de structure ;
    une unité d'estimation de catégories configurée pour estimer, sur la base de la au moins une valeur de caractéristique de tonalité de couleur ou valeur de caractéristique de structure calculée par l'unité de calcul de valeur de caractéristique, plusieurs catégories correspondant à chacune des zones extraites par l'unité d'extraction de zones de composantes de structure ;
    une unité d'estimation de paramètres configurée pour estimer, sur la base de la au moins une valeur de caractéristique de tonalité de couleur ou valeur de caractéristique de structure calculée par l'unité de calcul de valeur de caractéristique, des paramètres pour chacune des zones de l'image médicale classées respectivement dans les catégories estimées par l'unité d'estimation de catégories, les paramètres correspondant à la moyenne de la au moins une valeur de caractéristique de tonalité de couleur ou valeur de caractéristique de structure ; et
    une unité de segmentation d'image configurée pour réaliser une opération arithmétique se rapportant à la segmentation d'image en appliquant le nombre de catégories et les résultats d'estimation des paramètres à une expression de détection de contour d'un procédé de détection de contour dynamique sur la base d'un modèle de Mumford-Shah, réaliser une opération répétée en utilisant l'expression de détection de contour jusqu'à ce qu'une condition prédéterminée soit satisfaite, et réaliser le traitement de segmentation d'image pour l'image médicale sur la base d'un résultat de l'opération répétée lorsque la condition prédéterminée est satisfaite, **caractérisé par** les paramètres correspondant également à la déviation standard ou à la variance de la au moins une valeur de caractéristique de tonalité de couleur ou valeur de caractéristique de structure,
    dans lequel l'unité d'extraction de zones de composantes de structure est configurée pour réaliser les opérations suivantes :

        - créer une carte de bordure (M), en binarisant le résultat de filtrage de détection de bordure en utilisant une première valeur de seuil et en appliquant un étiquetage à chacune des composantes liées ;
        - créer une carte de bordure large (L) en binarisant le résultat de filtrage de détection de bordure en utilisant une deuxième valeur de seuil supérieure à la première valeur de seuil, d'où il résulte qu'une bordure large formant une zone fermée ou une bordure large contenue dans au moins une partie d'une zone fermée est spécifiée ;
        - sélectionner, parmi les composantes liées de la carte de bordure (M), les composantes liées qui chevauchent un pixel dans lequel il est déterminé qu'une bordure large de la carte de bordure large (L) est présente ;
        - extraire des zones fermées respectives formées par les composantes liées déterminées comme composantes liées comportant une bordure large en utilisant un procédé de détection de zone fermée pour obtenir des zones représentatives en tant que zones contenant diverses composantes de structure établies en tant que cibles pour l'unité de segmentation d'image ; et
        - définir, comme une zone d'arrière-plan globale, un groupe de pixels n'étant pas extraits en tant que bordure dans la carte de bordure (M).

**2.** Appareil (4) de traitement d'image médicale selon la revendication 1, dans lequel
l'unité d'estimation de catégories est configurée pour appliquer un traitement d'agrégation aux plusieurs catégories sur la base d'un état de répartition de la au moins une valeur de caractéristique de tonalité de couleur ou valeur de caractéristique de structure calculée par l'unité de calcul de valeur de caractéristique, et
l'unité de segmentation d'image est configurée pour définir un paramètre estimé pour chacune des zones respectivement classées dans des catégories estimées par un résultat de traitement du traitement d'agrégation dans l'unité d'estimation de paramètres.

**3.** Appareil (4) de traitement d'image médicale selon la revendication 1, dans lequel l'image médicale est une image endoscopique.

**4.** Appareil (4) de traitement d'image médicale selon la revendication 1, dans lequel au moins l'un parmi un vaisseau sanguin et une structure de creux est contenu dans les catégories estimées par l'unité d'estimation de catégories.

**5.** Appareil (4) de traitement d'image médicale selon la revendication 1, dans lequel l'unité de segmentation d'image est configurée pour déterminer que la condition prédéterminée est satisfaite, si une variation dans une valeur d'évaluation calculée en utilisant l'expression de détection de contour est sensiblement éliminée, ou si le nombre de répétitions de l'opération a atteint un nombre de fois prédéterminé.

**6.** Procédé de traitement d'image médicale destiné à réaliser un traitement de segmentation d'image pour une image médicale, le procédé exécutant :

une étape d'extraction de zones de composantes de structure dans laquelle une unité d'extraction de zones de composantes de structure extrait des zones correspondant à des composantes de structure contenues dans l'image médicale ;
une étape (S2) de calcul de valeur de caractéristique dans laquelle une unité de calcul de valeur de caractéristique calcule au moins une valeur de caractéristique de tonalité de couleur ou valeur de caractéristique de structure à partir de chacune des zones extraites par l'étape d'extraction de zones de composantes de structure ;
une étape (S3) d'estimation de catégories dans laquelle une unité d'estimation de catégories estime, sur la base de la au moins une valeur de caractéristique de tonalité de couleur ou valeur de caractéristique de structure calculée par l'étape de calcul de valeur de caractéristique, plusieurs catégories correspondant à chacune des zones extraites par l'étape d'extraction de zones de composantes de structure ;
une étape (S3) d'estimation de paramètres dans laquelle une unité d'estimation de paramètres estime, sur la base de la au moins une valeur de caractéristique de tonalité de couleur ou valeur de caractéristique de structure calculée par l'étape de calcul de valeur de caractéristique, des paramètres pour chacune des zones de l'image médicale respectivement classées dans les catégories estimées par l'étape d'estimation de catégories, les paramètres correspondant à la moyenne de la au moins une valeur de caractéristique de tonalité de couleur ou valeur de caractéristique de structure ; et
une étape (S4) de segmentation d'image dans laquelle l'unité de segmentation d'image réalise une opération arithmétique se rapportant à la segmentation d'image en appliquant le nombre de catégories et les résultats d'estimation des paramètres à une expression de détection de contour d'un procédé de détection de contour dynamique sur la base d'un modèle de Mumford-Shah, réalise une opération répétée en utilisant l'expression de détection de contour jusqu'à ce qu'une condition prédéterminée soit satisfaite, et réalise le traitement de segmentation d'image pour l'image médicale sur la base d'un résultat de l'opération répétée lorsque la condition prédéterminée est satisfaite,
**caractérisé par** les paramètres correspondant également à la déviation standard ou à la variance de la au moins une valeur de caractéristique de tonalité de couleur ou valeur de caractéristique de structure,
dans lequel l'étape d'extraction de zones de composantes de structure est définie pour réaliser les sous-étapes suivantes consistant à :

- créer une carte de bordure (M), en binarisant le résultat de filtrage de détection de bordure en utilisant une première valeur de seuil et en appliquant un étiquetage à chacune des composantes liées ;
- créer une carte de bordure large (L) en binarisant le résultat de filtrage de détection de bordure en utilisant une deuxième valeur de seuil supérieure à la première valeur de seuil, d'où il résulte qu'une bordure large formant une zone fermée ou une bordure large contenue dans au moins une partie de la zone fermée est spécifiée ;

- sélectionner, parmi les composantes liées de la carte de bordure (M), des composantes liées qui chevauchent un pixel dans lequel il est déterminé qu'une bordure large de la carte de bordure large (L) est présente ;
- extraire des zones fermées respectives formées par les composantes liées déterminées comme composantes liées comportant une bordure large en utilisant un procédé de détection de zone fermée pour obtenir des zones représentatives en tant que zones incluant diverses composantes de structure établies comme cibles pour l'étape de segmentation d'image ; et
- définir, en tant que zone d'arrière-plan globale, un groupe de pixels n'étant pas extraits en tant que bordure dans la carte de bordure (M).

7. Procédé de traitement d'image médicale selon la revendication 6, dans lequel
à l'étape d'estimation de catégories, un traitement d'agrégation est appliqué à la pluralité de catégories sur la base d'un état de répartition de la au moins une valeur de caractéristique de tonalité de couleur ou valeur de caractéristique de structure calculée par l'étape de calcul de valeur de caractéristique, et
l'étape de segmentation d'image définit un paramètre estimé pour chacune des zones respectivement classées dans des catégories estimées par un résultat de traitement du traitement d'agrégation à l'étape d'estimation de paramètres.

8. Procédé de traitement d'image médicale selon la revendication 6, dans lequel l'image médicale est une image endoscopique.

9. Procédé de traitement d'image médicale selon la revendication 6, dans lequel au moins l'un parmi un vaisseau sanguin et une structure de creux est contenu dans les catégories estimées par l'étape d'estimation de catégories.

10. Procédé de traitement d'image médicale selon la revendication 7, dans lequel l'étape de segmentation d'image détermine que la condition prédéterminée est satisfaite, si une variation dans une valeur d'évaluation calculée en utilisant l'expression de détection de contour est sensiblement éliminée, ou si le nombre de répétitions de l'opération a atteint un nombre de fois prédéterminé.

# FIG.1

# FIG.2

# FIG.3

# FIG.4

# FIG.5

```
            ┌─────────────┐
            │    START    │
            └─────────────┘
                   │
                   ▼
    ┌──────────────────────────────┐
    │          INPUT IMAGE         │──── S1
    └──────────────────────────────┘
                   │
                   ▼
    ┌──────────────────────────────┐
    │ CALCULATE COLOR-TONE FEATURE VALUE │──── S2
    └──────────────────────────────┘
                   │
                   ▼
    ┌──────────────────────────────┐
    │  ESTIMATE NUMBER OF CATEGORIES │
    │  AND PARAMETERS IN CALCULATION │──── S3
    │   OF CONTOUR DETECTING METHOD  │
    └──────────────────────────────┘
                   │
     ┌─────────────┤
     │             ▼
     │   ┌──────────────────────────────┐
     │   │   APPLY CONTOUR DETECTION     │──── S4
     │   │   EXPRESSION OF M-S METHOD    │
     │   └──────────────────────────────┘
     │             │
     │             ▼            S5
     │        ╱─────────────╲
     │       ╱ PREDETERMINED ╲
     │      ╱  NUMBER OF TIMES ╲      Y
     │      ╲   OF CALCULATION ╱─────────┐
     │       ╲   REACHED?     ╱          │
     │        ╲─────────────╱            │
     │             │ N                   │
     └─────────────┘                     │
                                         ▼
                              ┌─────────────┐
                              │     END     │
                              └─────────────┘
```

# FIG.6

# FIG.7

| 0 | 0 | −1 | 0 | 0 |
|---|---|---|---|---|
| 0 | 0 | 0 | 0 | 0 |
| −1 | 0 | 5 | 0 | −1 |
| 0 | 0 | 0 | 0 | 0 |
| 0 | 0 | −1 | 0 | 0 |

# FIG.8

# FIG.9

# FIG.10

EP 2 502 546 B1

# FIG.11

START

INPUT IMAGE — S11

CALCULATE COLOR-TONE FEATURE VALUE — S12

APPLY EM ALGORITHM WITH NUMBER OF CLASSES SET TWO — S13

ESTIMATE PARAMETERS — S14

CLASSIFY AREAS — S15

S16
DO ALL AREAS SATISFY PREDETERMINED CONDITIONS?
N
Y

ESTIMATE NUMBER OF CATEGORIES AND PARAMETERS IN CALCULATION OF CONTOUR DETECTING METHOD — S17

APPLY CONTOUR DETECTION EXPRESSION OF M-S METHOD — S18

S19
PREDETERMINED N UMBER OF TIMES OF CALCULATION REACHED?
Y
N

END

30

# FIG.12

MIXED NORMAL
DISTRIBUTION

COLOR-TONE FEATURE VALUE

# FIG.13

FIRST NORMAL
DISTRIBUTION

SECOND NORMAL
DISTRIBUTION

COLOR-TONE FEATURE VALUE

## REFERENCES CITED IN THE DESCRIPTION

### Patent documents cited in the description

- JP 2007313119 A
- US 2006050966 A1
- JP 11003428 A
- JP 2007175432 A

### Non-patent literature cited in the description

- Computer Vision Forefront Guide <1>. Advanced Communication Media Co., Ltd, 2009, 1-28
- **TONY F. CHAN ; LUMINITA A. VESE.** Active Contours Without Edges. *IEEE TRANSACTIONS ON IMAGE PROCESSING,* February 2001, vol. 10 (2), 266-277
- **CHRISTOPHE SAMSON ; LAURE BLANC-FERAUD ; GILLES AUBERT ; JOSIANE ZERUBIA.** Multiphase Evolution and Variational Image Classification. *INRIA Sophia Antipolis,* 1999
- Identification of polyps in Wireless Capsule Endoscopy videos using Log Gabor filters. **KARARGYRIS A et al.** LIFE SCIENCE SYSTEMS AND APPLICATIONS WORKSHOP. IEEE/NIH, 09 April 2009, 143-147
- **CHEOLHA PEDRO LEE.** Robust Image Segmentation using Active Contours: Level Set Approaches. *PhD Thesis - Dept. of Electrical and Computer Engineering North Carolina State University,* 01 June 2005, 1-146, URL:http://repository.lib.ncsu.edu/ir/bitstream/1840.16/5246/1/etd.pdf
- A Multiphase Level Set Framework for Image Segmentation Using the Mumford and Shah Model. **LUMINITA A VESE et al.** INTERNATIONAL JOURNAL OF COMPUTER VISION. KLUWER ACADEMIC PUBLISHERS, 01 December 2002, vol. 50, 271-293
- **CHRISTOPHE SAMSON ; LAURE BLANC-FERAUD ; GILLES AUBERT ; JOSIANE ZERUBIA.** Multiphase Evolution and Variational Image Classification. *INRIA Sophia Antipolis*
- Active Contours Without Edges. *IEEE TRANSACTIONS ON IMAGE PROCESSING,* February 2001, vol. 10 (2